(19) Europäisches Patentamt
European Patent Office
Office européen des brevets

(11) **EP 3 628 251 A1**

(12) **EUROPEAN PATENT APPLICATION**

(43) Date of publication:
**01.04.2020 Bulletin 2020/14**

(51) Int Cl.:
**A61B 18/02** (2006.01)     **A61B 18/04** (2006.01)
**A61B 18/18** (2006.01)     **A61B 34/10** (2016.01)

(21) Application number: **18197435.3**

(22) Date of filing: **28.09.2018**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Koninklijke Philips N.V.**
**5656 AG Eindhoven (NL)**

(72) Inventor: **HAUTVAST, Guillaume Leopold Theodorus Frederik**
**5656 AE Eindhoven (NL)**

(74) Representative: **de Haan, Poul Erik et al**
**Philips International B.V.**
**Philips Intellectual Property & Standards**
**High Tech Campus 5**
**5656 AE Eindhoven (NL)**

(54) **ABLATION THERAPY PLANNING SYSTEM**

(57)     It is an object of the invention to improve the patient safety during thermal ablation. This object is achieved by an ablation therapy planning system, configured to carry out the steps of: receiving a medical image of a patient, and receiving an input defining an intended treatment location for one or more thermal applicators relative to a skin location and one or more intended treatment parameters and determining a location of a skin of the patient and estimating a temperature or thermal dose at the skin location resulting from the intended treatment location and one or more intended treatment parameters and raising an alarm if the calculated temperature or thermal dose at the skin location is above or below a certain threshold.

FIG. 4B

## Description

FIELD OF THE INVENTION

The invention relates to the field of thermal ablation.

BACKGROUND OF THE INVENTION

Percutaneous thermal ablation is an interventional cancer treatment option that has seen significant increase in adoption in the past decade. Thermal ablation can be delivered using various ablation modalities, including radiofrequency (RF), microwave (MW), high-intensity focussed ultrasound (HIFU), focal laser ablation (FLA), irreversible electroporation (IRE), cryo-ablation, etc.

In clinical practice, these ablation procedures consist of placing one or more ablation applicators inside or near the target region with the help of image guidance. Typically, clinicians place these needle-like applicators while inspecting real-time ultrasound or interventional radiology images (CT/MR), based on information provided from the manufacturer, results in clinical trials and personal experience. The use of more advanced ablation therapy planning systems (ATPS) to plan the ablation and guide needle placement, akin to the radiation therapy planning systems (RTPS) used in brachytherapy procedures, is not wide spread due to their limited availability.

A clinical thermal ablation treatment is very complex, relying on a combination of clinical procedures that all need to be monitored closely to guarantee optimal treatment outcome. Next to the placement of the ablation applicators and performing the ablation, this includes:

- intra-operative (real-time) imaging,
- anesthesia for the patient,
- the placement of temperature probes,
- the placement of urethral catheters,
- the placement of warmers/coolers to protect organs at risk (OAR),
- performing a hydro/pneumo dissection to protect the OAR.

US 2016/0335413 A1 describes a system and method for minimally invasive thermal ablation treatment planning. The described system is configured for analyzing a thermal dose distribution to determine treatment effectiveness.

SUMMARY OF THE INVENTION

It is an object of the invention to improve the patient safety during thermal ablation. This object is achieved by an ablation therapy planning system according to a claim 1.

Because the clinicians performing the thermal ablation treatment need to distribute their attention to monitor all aspects of the treatment, adverse effects are sometimes detected late, or even go completely undetected until after completion of the procedure. These adverse effects may be caused by accidently incorrect positioning or choosing the incorrect treatment parameters. In addition, it appears to be complicated to oversee how changes in the applicator positioning and ablation parameters at the treatment site may affect temperature at the skin. Consequently, skin irritation and/or skin burns are reported adverse events of thermal ablation procedures. By applying a thermal model, the temperature at the skin location can be predicted prior to application of the ablation. By raising an alarm when the temperature of the skin is predicted to get too warm (e.g. when heating the target) or too cold (e.g. when cooling the skin) adverse skin effects may be prevented and patient safety may be improved.

The skin location may be directly derived from the medical image, e.g. when using segmentation. However, the skin may not be visible in all images from all imaging modalities. For example, when using an endorectal ultrasound probe, the skin location will most likely not be visible in the ultrasound image. In these situations, the skin location may be determined by performing image registration of the medical image with a second medical image of the same patient and target, wherein the second medical image is acquired by means of a different imaging modality than the medical image. This second medical image could for example be a CT or MRI image. This is advantageous, because in many occasions a CT or MRI images of the patient are acquired anyway for determination of the location of the target. For example, prostate cancer is hard to locate by means of ultrasound images alone. Therefore, an MRI and / or (contrast-enhanced) CT image may be acquired for that purpose. When the location of the skin can be derived from the second medical image, image registration with the ultrasound image transforms the coordinates of the skin in the second medical image to coordinates in the ultrasound image space.

Alternatively or additionally, the location of the skin can be determined by means of a device having a known position relative to the skin location. The device could for example be placed onto the skin, but could also for example have a fixed position relative to the skin. The position of the device can be determined by means of tracking and / or could be determined by means of a pre-procedure calibration, in which the location of the skin relative to the coordinates of the target is determined.

According to embodiments of the invention, the ablation therapy planning system is configured for calculating isotherms or isodose lines, wherein the ablation therapy planning system is configured for raising an alarm when predefined isotherms or isodose lines are crossing the skin location or are within a predefined distance from the skin location. Thermal dose reflects temperature integrated over time and can for example be expressed in equivalent number of minutes at 42 or 43°C.

Thermal dose may in some cases provide for a better prediction of skin damage than temperature.

According to embodiments of the invention, the treatment parameters are a fixed set of parameter configurations to encode different ablation zones, isotherms or isodoses is used to estimate the temperature or thermal dose at the skin location. These configurations may describe the size and shape of the ablation zone or different isotherm or isodose lines. This embodiment is advantageous, especially during treatment, because it can reduce computation time.

Alternatively, thermal models to estimate temperature of thermal dose at the location of the skin can be used. Such models can be based on literature or average values for tissue parameters. The values can be improved by stratification of patient groups based on for example age, gender and / or specific medical conditions. Also the values of some of the parameters may be measured for the individual patient by means of medical imaging, for example MRI. Such medical images may for example provide information about vessel density.

According to embodiments of the invention settings of the alarm are configurable by a user. For example, the user may select a skin temperature at which the alarm is raised or a distance from an isodose or isotherm line. This embodiment is advantageous because it increases the flexibility of the system to adapt to different treatment types, but also to adapt to user preferences or latest scientific insights with respect to temperature induced skin toxicity.

The ablation therapy planning system according to embodiments of the invention can be used during the treatment planning stage. During this stage, based on the location and size of the target and the location nearby organs at risk a treatment plan is created for the patient. Such plan may comprise intended treatment target locations for one or more percutaneous thermal applicators to be used during treatment. The ablation therapy planning system according to embodiments of the invention can be used in this planning phase to warn the user for potential skin damage when a calculated treatment plan will be executed.

Alternatively or additionally, the ablation therapy planning system may be used during the actual ablation treatment, i.e. during any of the step including and between applicator positioning and treatment delivery. After the clinician has inserted the treatment device, the location of the thermal applicator may be detected (semi)automatically by the ablation therapy planning system, but also may be inserted into the system by a user. Based on this location, the ablation therapy planning system may perform temperature calculations and predict the temperature at the skin when using the current thermal applicator position in combination with the intended treatment parameters. These temperature calcualtions may include the locations and treatment parameters of previously inserted applicators and may further include the intended locations and treatment parameters for thermal applicators that still have to be inserted, if the latter information is available. The alarm will be raised if the calculated temperature at the skin location is above or below a certain threshold. Using the ablation therapy planning system during the actual ablation treatment provides for an extra safety measure against accidently incorrect applicator placement, causing skin damage. However, also it may help during procedures, wherein the clinician decides during the procedure to deviate from the plan. When deviating from the plan, the user may not always be aware of the potential skin effects. Raising the alarm may therefore improve the safety.

When an approved treatment plan is available, it may not always be necessary to perform the temperature calculations once an applicator has been inserted during treatment, because it is known that if the treatment is executed according to plan, no safety issues are to be expected. However, this situation changes if the situation has changed compared to the planning stage. For example, the patient anatomy may have changed, but also the applicator(s) may not have been as accurately positioned as assumed. Therefore, according to embodiments of the invention, the skin temperature calculation is only performed when the location of the one or more thermal applicator or the one or more treatment parameters is changed more than a predetermined value. This embodiment is advantageous, because calculations during treatment may slow down the treatment process.

These and other aspects of the invention will be apparent from and elucidated with reference to the embodiments described hereinafter.

BRIEF DESCRIPTION OF THE FIGURES

Fig. 1 diagrammatically shows a clinical workflow in which embodiments of the invention can be used and Fig. 2 diagrammatically shows an ablation therapy planning system according to embodiments of the invention and
Fig. 3 diagrammatically shows a cryoablation needle and the corresponding isotherm lines and
Fig. 4 diagrammatically illustrates a situation wherein the alarm would not and would be raised and
Fig. 5 diagrammatically illustrates another situation wherein the alarm would not and would be raised and
Fig. 6 diagrammatically illustrates how devices can be used to determine the position of the skin.

DETAILED DESCRIPTION OF THE INVENTION

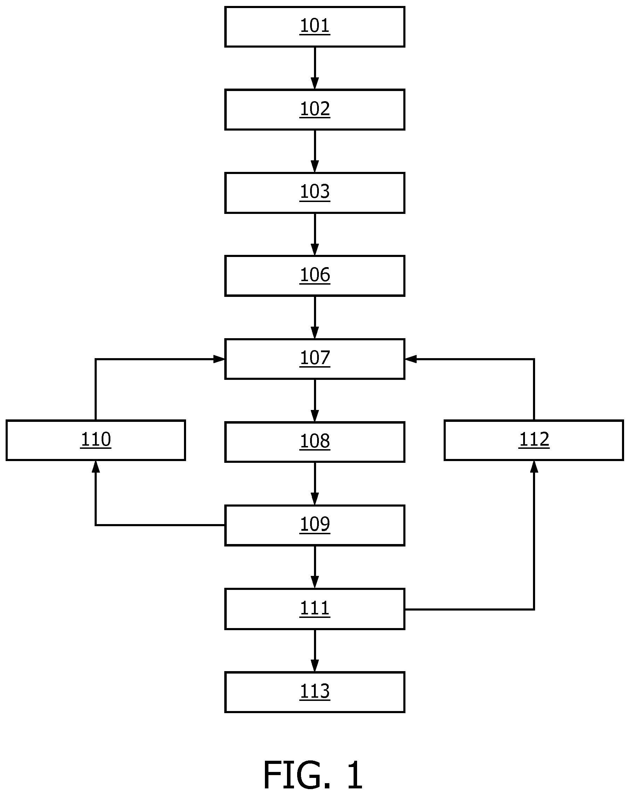

Fig. 1 diagrammatically shows a clinical workflow in which embodiments of the invention can be used. One or more medical images (e.g. CT or MRI) maybe acquired to identify a treatment target in a patient that is or may be scheduled for ablation treatment. Such medical image may be referred to below as second medical image. This medical image may be used to segment the target for treatment (e.g. a tumor including a certain uncertainty margin) 101. Also, one or more organs at risk may be segmented 101. This segmentation may be performed manually or (semi-)automatically.

Prior to starting the treatment, the patient will be positioned on the treatment table. Also, the devices to be used during the treatment may be set up and calibrated. Image guidance could for example be achieved by means of ultrasound imaging, but other medical imaging modalities are also possible. Image registration to the second medical image can be used to determine the location of the skin relative to the ultrasound (or other imaging modality) imaging frame and possibly also for determination of the position of the target. By means of the image registration output, the coordinates of the skin and target in the second medical image can be transformed to coordinates in the image space of the image used during the ablation procedure.

Also, the skin may be directly derived from the medical images used during the ablation procedure. An example of an implementation where the skin location is derived from medical images, would be in MR/CT-guided interventional radiology procedures on the liver or kidney. In this case, the skin can easily be automatically located at the boundary of the patient torso and the surrounding air in the bore.

Also, the location of the skin may be derived from devices placed onto the skin or placed on a fixed distance from the skin in order to determine the location of the skin relative to the target area and the one or more applicators. This could for example be achieved by means of tracked devices. An example of an implementation where the skin location is derived from a tracked device placed against the skin, could be in treatments supported by a patient tracker. Such patient trackers are used to track patient motion, but can also be used for the purpose of skin protection alarm as described in this invention. The Philips PercuNav system includes such a patient tracker.

Further, the skin location relative to the target and applicator position could be determined by the use of devices with a more or less fixed position. An example would be in urology interventions in which a grid template is placed against the patients' perineum. In this way the skin position is known through pre-procedure calibration.

Even further, the skin location can be determined directly, e.g. by means of one or more cameras. A calibration may be needed to relate the position of the skin determined with one or more cameras to the position of the target determined by means of the medical imaging system.

Based on the segmented target and one or more organs at risk a treatment plan can be created 102. The creation of the treatment plan may be an optimization of therapeutic effect, wherein therapeutic effect is defined by a combination of likelihood to successfully treat the target, while keeping toxicity levels within certain limits.

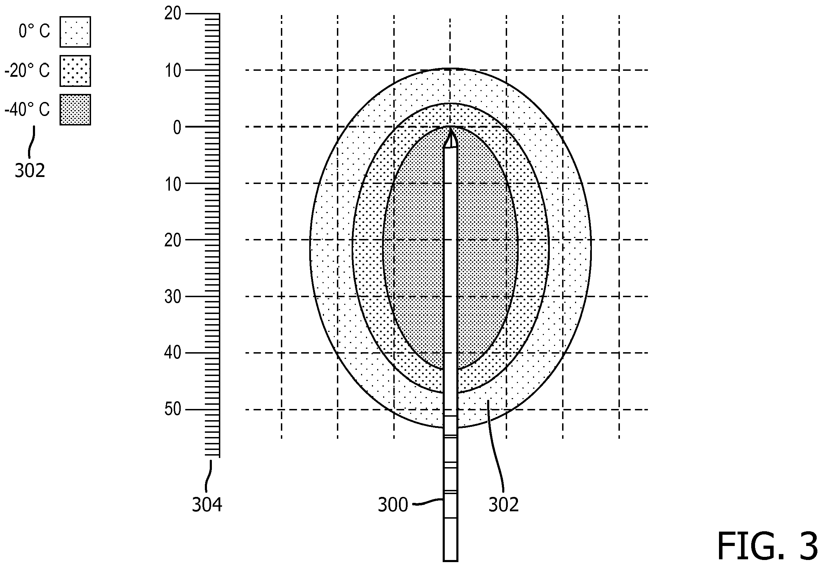

The result of the treatment plan will be the location(s) of one or more treatment applicators and the treatment parameters to be used for each applicator. These treatment parameters could for example be duration of heating or cooling, power used or a direction in which the treatment is provided. These treatment parameters could also be the shape of isotherm or isodose lines relative to the applicator, as will be explained in figure 3. The location(s) of the one or more applicators in combination with their treatment parameters will result in a certain temperature distribution during the actual treatment delivery. This temperature distribution may be estimated by means of a thermal model. This temperature distribution may also affect the skin. Both too high and too cold skin temperatures may cause skin damage. Also the duration of the exposure of the skin to certain temperatures may be important. The temperature distribution can be used to estimate the skin temperature during the actual treatment 103. If the temperature at the skin is estimated to be too high or too low (depending on whether the treatment will be a heating or cooling treatment), an alarm will be raised. This alarm notifies the user that alterations to the plan are likely to be needed. Also, the user may consider the use of countermeasures that counteract the temperature changes at the skin resulting from the treatment. For example external skin cooling may be applied.

Once the planned applicator positions are calculated, these planned positions may be projected onto the medical image. Further, information may be provided to the clinician on how to access these locations, for example in procedures targeting the prostate an insertion hole in the grid may be indicated. This will assist the clinician to place the applicator at the correct position 107. However, in practise, the clinician may want to deviate from the plan, for example based on the real-time imaging. Also, the applicators may not be placed exactly as planned. This may result in a different temperature distribution and therefore also affect the temperature at the skin. After applicator positioning the temperature distribution may be recalculated or only be recalculated if the applicator position deviates more than a certain value from the planned position 108. The temperature distribution may be calculated by using the positions of the already placed applicators. Also the positions of the remaining applicators could be taken into account by assuming that they will be positioned at their planned location. Optionally, some error margin for placing the remaining applicators could be taken into accound. Based on the recalculated temperature distribution, the estimation of the skin temperature as a result of the treatment may be repeated 109. If the estimated skin temperature is above or below (for heating and cooling treatments respectively) a certain threshold the alarm will be raised 110 and the system will suggest the clinician to reposition the applicator 107 or change the treatment parameters. If no alarm is raised, the clinician will assess if all applicators have been positioned 111. If not 112, the next applicator will be placed 107. Once all applicators have been placed and no (remaining) risk for skin damage is determined, thermal treatment can be started 113.

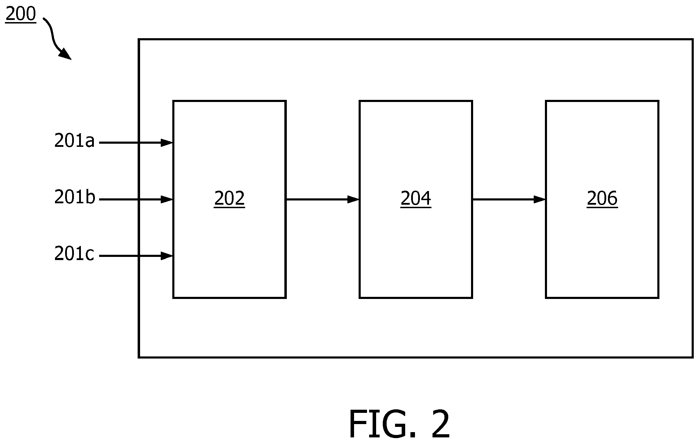

Fig. 2 diagrammatically shows an ablation therapy planning system 200 according to embodiments of the invention. The ablation therapy planning system receives a medical image of a patient 201a. In addition it receives an input defining an intended treatment location for one or more thermal applicators 201b relative to a skin location. This intended treatment location could either be a planned location or the location of an applicator that has been placed for ablation treatment. The ablation therapy planning system also receives one or more intended treatment parameters 201c. Based on the medical image a location of a skin of the patient 202 relative to the target or intended treatment location can be determined. This may be derived directly from the medical image, but as explained above, alternative methods are possible. Then the system uses a thermal model to estimate the temperature at the skin location from the intended treatment location and one or more intended treatment parameters 204.

For example such thermal model could comprise three elements:

- A heat equation describing the temperature evolution in time given a heat source
- An equation (or a set of equations) describing the application modality and deriving the appropriate heat source for the heat equation.
- An additional equation (or a set of equations) translating the temperature evolution into a thermal dose, i.e. into a prediction of the probability of cell thermal damage. These equations are generally coupled because of the strong dependence of the properties of tissue on temperature and cell damage probability.

The temperature evolution can be modelled using a classic Pennes equation (so perfusion is modeled as a distributed perfusion rate $\omega_b$), in which the heat source (or heat sink) distribution for the therapy device is modeled with a source term Q:

$$\rho C_p \frac{\partial T}{\partial t} = \nabla \cdot \lambda \nabla T + \omega_b \rho_b C_b (T_b - T) + Q$$

, where $Cp$ is the heat capacity (including the specific heat of fusion at 0°), $\rho$ is the density and $\lambda$ is the thermal conductivity. The second term at the right hand side is the perfusion term with the suffix $b$ standing for blood. Perfusion is modeled as a distributed perfusion rate $\omega b$. $T_b$ is the temperature of the blood and $T$ the temperature of the surrounding tissue.

Within this model, the perfusion rate in space may be uniform or non-uniform, e.g. higher inside vessels as visible in the acquired image. This equation is then integrated in time to derive the temperature field that will be fed to the cell damage model.

The thermal equation can be solved with different numerical method, including the Finite Difference Time Domain (FDTD) and Finite Element Method (FEM) approaches. In the Finite Element Approach, the weak form of the partial differential equations associated with the ablation are discretized and subsequently solved directly or iteratively.

In the FDTD approach, the time-dependent equations in partial differential form are discretized using central-difference approximations to the space and time partial derivatives. The resulting finite-difference equations are solved using a leapfrog integration method.

As an alternative to the thermal model a fixed set of parameter configurations to encode the ablation zone of the applicator may be used in order to estimate skin temperature or thermal dose at the skin.

Based on the temperature calculations made by the thermal model, the system raises an alarm if the calculated temperature at the skin location is above or below a certain threshold 206.

Fig. 3 diagrammatically shows a cryoablation needle 300 and the corresponding isotherm lines 302. 304 is a scale to show the height of the ablation zone in millimeters. Figure 3 displays an -40°C isotherm line, which is closest to the applicator. Further, an -20°C isotherm (middle) and 0°C isotherm line are displayed. This information about the location of isotherm lines is provided for almost all the ablation needles available. This information can be used by the ablation therapy planning system as an alternative to the thermal model to determine whether the alarm should be raised.

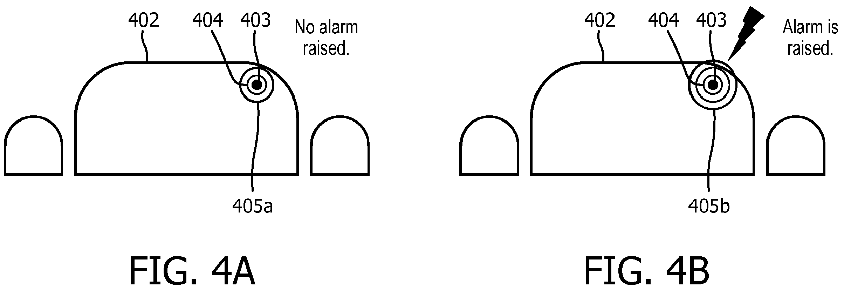

Fig. 4 diagrammatically illustrates a situation wherein the alarm would not (A) and would (B) be raised. Figure 4 diagramatically illustrates a patient in which an applicator is placed 203. As a result of the applicator use, local temperature will change. This can be seen by the presence of isodose or isotherm lines 404, 405. In the implementation according to figure 4, the alarm will be raised if a certain isodose or isotherm line 405 intersects with the skin 402. In figure 4A, isodose line 405a does not intersect with the skin 402, so no alarm will be raised. In figure 4B, isodose line 405 does intersect with the skin 402. Hence the alarm will be raised.

According to embodiments of the invention, the alarm could be configurable by the user. For example the user may specify a distance for any region (ablation zone, specific isotherm or specific isodose line). For example, the alarm maybe raised if the 50 degree isotherm line is closer than 5 mm to the skin.

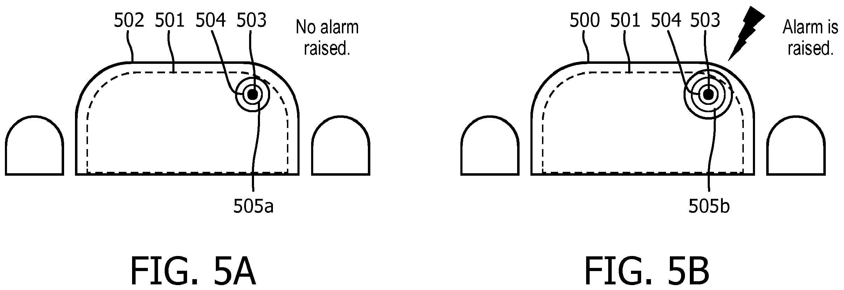

Fig. 5 diagrammatically illustrates another situation wherein the alarm would not A and would B be raised. In this implementation the alarm will be raised if the isodose or isotherm line 505 is within a certain distance from the skin 502. 501 shows the line have this certain distance from the skin 502. In figure 5A the alarm will not be raised, because the isodose line 505a is not intersecting with line 501. In figure 5B, line 505b is intersecting with 501. Hence, the alarm will be raised.

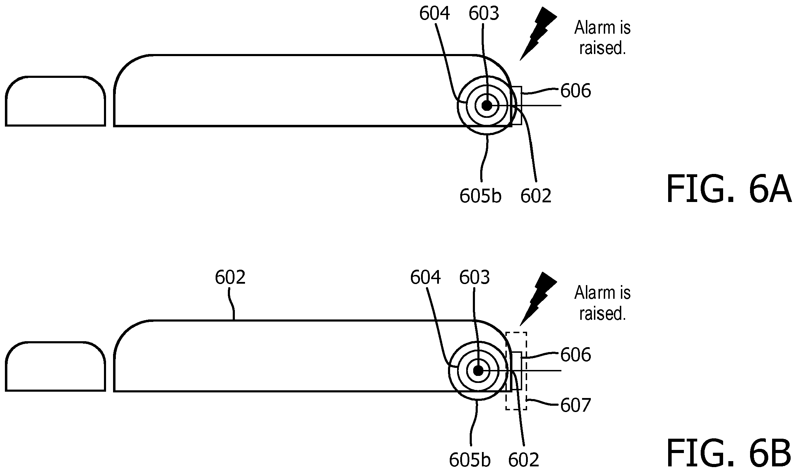

Fig. 6 diagrammatically illustrates how devices can be used to determine the position of the skin. Figure 6 diagrammatically displays a prostate procedure, wherein a grid 606 is positioned close to the skin 602 of the perineum. The grid is taken as a surrogate for the skin location 602. An alarm is raised when isodose line 605 intersects with the grid (figure 6A) or when the isodose line 605 is within a certain distance from the grid 607 (figure 6B).

Whilst the invention has been illustrated and described in detail in the drawings and foregoing description, such illustrations and description are to be considered illustrative or exemplary and not restrictive; the invention is not limited to the disclosed embodiments.

**Claims**

1. An ablation therapy planning system, comprising program code means for causing the ablation therapy planning system to carry out the steps of:

   - receiving a medical image of a patient, and receiving an input defining an intended treatment location for one or more thermal applicators relative to a skin location and one or more intended treatment parameters and;
   - determining a location of a skin of the patient and;
   - estimating a temperature or thermal dose at the skin location resulting from the intended treatment location and one or more intended treatment parameters and;
   - raising an alarm if the calculated temperature or thermal dose at the skin location is above or below a certain threshold.

2. An ablation therapy planning system as claimed in claim 1, wherein the treatment parameters are a fixed set of parameter configurations to encode different ablation zones, isotherms or isodoses.

3. An ablation therapy planning system as claimed in claim 2, wherein the ablation therapy planning system is configured for raising an alarm when predefined isotherms or isodose lines are intersecting the skin location or are within a predefined distance from the skin location.

4. An ablation therapy planning system as claimed in claim 1, wherein the temperature at the skin is estimated by means of a thermal model.

5. An ablation therapy planning system as claimed in any of the preceding claims, wherein the skin location is derived from registration of the medical image with a second medical image of the same patient and target, wherein the second medical image is acquired by means of a different imaging modality than the medical image.

6. An ablation therapy planning system as claimed in any of the preceding claims, wherein the skin location relative to the one or more thermal applicators can be derived from a location of a device having a known position relative to the skin location, wherein the position is known through the use of a tracking system or a pre-procedure calibration.

7. An ablation therapy planning system as claimed in any of the preceding claims configured for use during the ablation treatment

8. An ablation therapy planning system as claimed in claim 7, configured for repeating the skin temperature calculation when the location of the one or more thermal applicator or the one or more treatment parameters is changed more than a predetermined value.

9. An ablation therapy planning system as claimed in any of the preceding claims 7-8, wherein the skin temperature is partly based on temperature measurements.

10. An ablation therapy planning system as claimed in any of the preceding claims 4-10, wherein the skin temperature is calculated using a patient specific thermal model.

11. An ablation therapy planning system as claimed in any of the preceding claims, wherein the alarm settings are configurable by a user.

FIG. 1

FIG. 2

0° C

-20° C

-40° C

302

20

10

0

10

20

30

40

50

304

300

302

FIG. 3

Alarm is raised.

402 404 403

405b

FIG. 4B

No alarm raised.

402 404 403

405a

FIG. 4A

502 501 504 503  No alarm raised.

505a

**FIG. 5A**

500 501 504 503  Alarm is raised.

505b

**FIG. 5B**

EP 3 628 251 A1

604 603

Alarm is raised.

606

605b 602

FIG. 6A

602

604 603

Alarm is raised.

606

607

605b 602

FIG. 6B

EP 3 628 251 A1

**Europäisches Patentamt**
**European Patent Office**
**Office européen des brevets**

# EUROPEAN SEARCH REPORT

Application Number

EP 18 19 7435

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2010/185087 A1 (NIELDS MORGAN W [US] ET AL) 22 July 2010 (2010-07-22) * the whole document * | 1-11 | INV.<br>A61B18/02<br>A61B18/04<br>A61B18/18<br>A61B34/10 |
| A | WO 2004/007020 A2 (CELSION CORP [US]; FENN ALAN J [US]; MON JOHN [US]) 22 January 2004 (2004-01-22) * abstract; claim 1 * * paragraph [0076] * | 1-11 | |
| A | WO 2010/052596 A1 (KONINKL PHILIPS ELECTRONICS NV [NL]; DALAL SANDEEP [US]; RAJU BALASUND) 14 May 2010 (2010-05-14) * the whole document * | 1-11 | |
| A | WO 2011/070476 A1 (KONINKL PHILIPS ELECTRONICS NV [NL]; MIELEKAMP PIETER M [NL]) 16 June 2011 (2011-06-16) * the whole document * | 1-11 | |

| | | | TECHNICAL FIELDS SEARCHED (IPC) |
|---|---|---|---|
| | | | A61B |

The present search report has been drawn up for all claims

| Place of search | Date of completion of the search | Examiner |
|---|---|---|
| Munich | 27 March 2019 | Beck, Ewa |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 18 19 7435

This annex lists the patent family members relating to the patent documents cited in the above-mentioned European search report.
The members are as contained in the European Patent Office EDP file on
The European Patent Office is in no way liable for these particulars which are merely given for the purpose of information.

27-03-2019

| Patent document cited in search report | | Publication date | Patent family member(s) | | Publication date |
|---|---|---|---|---|---|
| US 2010185087 | A1 | 22-07-2010 | NONE | | |
| WO 2004007020 | A2 | 22-01-2004 | AU | 2003253877 A1 | 02-02-2004 |
| | | | CA | 2491924 A1 | 22-01-2004 |
| | | | CN | 1668359 A | 14-09-2005 |
| | | | EP | 1523370 A1 | 20-04-2005 |
| | | | JP | 2005532868 A | 04-11-2005 |
| | | | MX | PA05000565 A | 28-04-2005 |
| | | | US | 2002193849 A1 | 19-12-2002 |
| | | | WO | 2004007020 A2 | 22-01-2004 |
| WO 2010052596 | A1 | 14-05-2010 | BR | PI0914520 A2 | 15-12-2015 |
| | | | CN | 102202735 A | 28-09-2011 |
| | | | EP | 2352558 A1 | 10-08-2011 |
| | | | JP | 5948057 B2 | 06-07-2016 |
| | | | JP | 2012507324 A | 29-03-2012 |
| | | | RU | 2011122671 A | 20-12-2012 |
| | | | US | 2011208055 A1 | 25-08-2011 |
| | | | WO | 2010052596 A1 | 14-05-2010 |
| WO 2011070476 | A1 | 16-06-2011 | BR | 112012013557 A2 | 10-10-2017 |
| | | | CN | 102647956 A | 22-08-2012 |
| | | | EP | 2509527 A1 | 17-10-2012 |
| | | | JP | 6035148 B2 | 30-11-2016 |
| | | | JP | 2013512748 A | 18-04-2013 |
| | | | RU | 2012128523 A | 20-01-2014 |
| | | | US | 2012237105 A1 | 20-09-2012 |
| | | | WO | 2011070476 A1 | 16-06-2011 |

EPO FORM P0459

For more details about this annex : see Official Journal of the European Patent Office, No. 12/82

**REFERENCES CITED IN THE DESCRIPTION**

**Patent documents cited in the description**

- US 20160335413 A1